# EUROPÄISCHE PATENTSCHRIFT

(11) **EP 1 007 060 B1**
(45) Veröffentlichungstag und Bekanntmachung des Hinweises auf die Patenterteilung: **23.11.2005**
(21) Anmeldenummer: 98929214.9
(22) Anmeldetag: 07.04.1998
(51) Int. Cl.: A61K 35/00, A61P 25/24

(54) **STABILER EXTRAKT AUS HYPERICUM PERFORATUM L., VERFAHREN ZU SEINER HERSTELLUNG UND PHARMAZEUTISCHE ZUBEREITUNGEN**
STABLE EXTRACT OF HYPERICUM PERFORATUM L., A METHOD FOR PRODUCING THE SAME, AND CORRESPONDING PHARMACEUTICAL PREPARATIONS
EXTRAIT STABLE D'HYPERICUM PERFORATUM L., SON PROCEDE DE PRODUCTION ET PREPARATIONS PHARMACEUTIQUES LE CONTENANT

(30) Priorität: 08.04.1997 DE 19714450
(43) Veröffentlichungstag der Anmeldung: 14.06.2000
(73) Patentinhaber: Dr. Willmar Schwabe GmbH & Co. KG, 76227 Karlsruhe (DE)
(72) Erfinder: ERDELMEIER, Clemens, D-76139 Karlsruhe (DE); GRETHLEIN, Eckhart, D-76327 Pfinztal (DE); LANG, Friedrich, D-76767 Hagenbach (DE); OSCHMANN, Rainer, D-76829 Landau (DE); STUMPF, Karl-Heinz, D-76228 Karlsruhe (DE)
(74) Vertreter: Adam, Holger
(86) Internationale Anmeldenummer: PCT/DE1998/001003
(87) Internationale Veröffentlichungsnummer: WO 1998/044936

(56) Entgegenhaltungen:
- WO-A-97/13489

## Beschreibung

Durch pharmakologische und klinische Versuche ist belegt, daß Extrakte aus Johanniskraut (Hypericumextrakte) bei depressiven Verstimmungszuständen bis hin zu mittelschweren Depressionen mit Erfolg eingesetzt werden können. Die milde antidepressive Gesamtwirkung konnte jedoch noch nicht eindeutig einem oder mehreren Inhaltsstoffen zugeordnet werden; vgl. J. Hölzl, S. Sattler und H. Schütt, Johanniskraut: eine Alternative zu synthetischen Antidepressiva, *Pharmazeutische Zeitung* Nr. 46, 139. Jahrgang, 17. November 1994, 3959-3977 und E. Steinegger und R. Hänsel, Pharmakognosie, 5. Aufl. 1992, S. 672-674, Springef Verlag. Allerdings gibt es gerade in neuester Zeit verstärkt Hinweise, daß zur Erzielung der Wirksamkeit Hyperforin wesentlich beiträgt (EP-A-0 599 307).

Die Arzneidroge besteht aus den oberirdischen Teilen von Hypericum perforatum L. Die Inhaltsstoffe von Hypericum perforatum L. sind unter anderem Hypericin und Hyperforin; vgl. J. Hölzl et al., a.a.O.

Die Herstellung von Hypericumextrakten mit angereichertem Hypericin-Gehalt ist in der DE-PS 1 569 849 sowie von S. Niesel und H. Schilcher in *Arch. Pharm.,* Bd. 323 (1990), 755 beschrieben.

Aus der Arbeit von R. Berghöfer und J. Hölzl, *Deutsche Apothekerzeittung,* Bd. 126, Nr. 47 (1986), Seiten 2569 - 2573, ist bekannt, daß Hyperforin in Extrakten aus gelagerter Droge bereits nach einer Woche völlig abgebaut ist, während es im Frischpflanzenextrakt stabiler sein soll. Die Verfasser vermuten, daß Frischpflanzen einen Stabilisator für Hyperforin enthalten.

J. Hölzl et al., *Planta Med*., Bd. 55 (1989), Seiten 601 - 602, berichten über Hypericumöl und einen vermuteten Zusammenhang zwischen der Hypericinkonzentration und der Peroxidzahl (POZ) Dem Sonnenlicht ausgesetzte Hypericumölpräparate zeigen unterschiedliche Peroxidzahlen. Nach J. Hölzl et al. besteht aber kein Zusammenhang zwischen dem POZ-Wert und der Hypericinkonzentration.

Über die Stabilität von Hypericumöl berichten P. Maisenbacher und K.-A. Kovar in *Planta Med*., Bd. 58 (1992), Seiten 351 - 354. Dieses Öl enthielt auch Hyperforin, das innerhalb weniger Wochen zersetzt war.

Die EP-A-0 599 307 beschreibt Primärextrakte aus Johanniskraut-Droge (vgl. Beispiele 1-3). Diese Primärextrakte werden durch einfache Extraktion der Droge mit 96%igem bzw. 60%igem wäßrigen Ethanol ohne irgendwelche Vorsichtsmaßnahmen und ohne irgendwelche Zusätze erhalten.

In Beispiel 1 ist der Hyperforingehalt des [frischen] Primärextraktes mit 6,3% angegeben, in den beiden anderen Beispielen sind keine Hyperforingehalte genannt. Wir haben festgestellt, daß derartige Hyperforinextrakte extrem instabil sind; vgl. Beschreibung, Beispiel 5, Tabelle 1. Der gemäß Beispiel 1 (Vergleich) durch Extraktion der Droge mit 70%igem wäßrigen Ethanol erhaltene Primärextrakt enthält nach 13 Wochen keine nachweisbaren Mengen an Hyperforin.

Ferner ist bekannt, Hypericumöl (Johannisöl, Oleum hyperici) durch Extraktion von zerquetschten frischen Johanniskrautblüten mit einem fetten Öl wie Olivenöl, Sojaöl, Weizenkeimlingsöl oder Sonnenblumenöl herzustellen. Hypericumöl enthält variable Mengen an Hyperforin und eignet sich zur äußerlichen Behandlung von Wunden, insbesondere Brandwunden und Verschürfungen; vgl. P. Maisenbacher und K.-A. Kovar, *Planta Med*., Bd. 58 (1992), 351 - 354 und J. Hölzl, L. Demisch und S. Stock, *Planta Med*., Bd. 55 (1989), 601 - 602.

Sowohl in der Droge als auch in üblichen Hypericumextrakten nimmt der Hyperforingehalt bei normaler Lagerung innerhalb weniger Monate drastisch ab bis zum Verschwinden der Substanz; vgl. Dissertation von P. Maisenbacher, Tübingen 1991, und Dissertation von R. Berghöfer, Marburg/L. 1987. In früheren Versuchen mit öligen Hypericumextrakten konnte die Stabilität hyperforinhaltiger Zubereitungen lediglich durch Lagerung unter Argon deutlich verbessert werden; vgl. Dissertation von P. Maisenbacher, Tübingen 1991. Eine Stabilisierung durch Antioxidantien wie Butylhydroxytoluol (BHT) und Butylhydroxyanisol (BHA) gelang in diesen Extrakten nicht. Desgleichen bringen übliche Antioxidantien wie Oxynex LM und Oxynex 2004 keine Verbesserung der Stabilität. Bei Hypericumöl wird nach P. Maisenbacher, Dissertation, die beste Stabilität durch Verwendung von Octyldodecanol (Eutanol G) als Extraktionsmittel erreicht. Auf Seite 154-155 gibt Maisenbacher historische Herstellungsvorschriften. Die Droge wird mit heißem Pflanzenöl, manchmal unter Zusatz von Terpentinöl, extrahiert. Auch hier beobachtete Maisenbacher Zersetzung, insbesondere unter dem Einfluß von Wasser. Auf Seite 158-166 schildert Maisenbacher die Bedingungen für ein optimiertes Herstellungsverfahren. Folgende Anweisungen sind besonders aufschlußreich: Wasser ist fernzuhalten; Verwendung von Eutanol G als Extraktionsmittel; übliche Antioxidantien gewähren keinen Oxidationsschutz. Nach einer Induktionsperiode von drei Wochen begann beim Olivenöl-Ansatz ein stetiger Abbau der Hyperforine; vgl. Abb. 7-10. Trotz Schutzbegasung war eine vollständige Zersetzung nach drei Monaten eingetreten; vgl. Seite 160. Das mit Eutanol G hergestellte Öl ist über ein halbes Jahr bei Raumtemperatur unter Argon stabil, bei 30°C erfolgt langsamer Abbau; vgl. Seite 161.

Hyperforinhaltige Hypericumextrakte können mit in der Pharmazie üblichen anorganischen oder organischen Lösungsmitteln oder deren Gemischen hergestellt werden (P. List und P.C. Schmidt, Technologie pflanzlicher Arzneizubereitungen, Wissensch. Verlagsgesellschaft mbH, Stuttgart 1984)

In üblichen ethanolisch-wässrigen Hypericumextrakten und daraus hergestellten Fertigarzneimitteln ist, bezogen auf den Extrakt, in der Regel weniger als ca. 1 % Hyperforin enthalten. Nach Lagerung sinkt der Wert deutlich ab und geht je nach Lagerungsbedingungen gegen null. Man vermutet, daß Oxidationsprozesse für den Abbau des Hyperforins in der Droge und im Extrakt verantwortlich sind.

Der Erfindung liegt die Aufgabe zugrunde, Hyperforin enthaltende stabilisierte Extrakte aus Hypericum perforatum L. (Johanniskraut) bereitzustellen, in denen das Hyperforin stabil bleibt. Eine weitere Aufgabe der Erfindung ist es, Verfahren zur Herstellung dieser stabilisierten Extrakte sowie diese enthaltende Arzneimittel bereitzustellen, in denen der Hyperforingehalt ebenfalls stabil bleibt.

Diese Aufgaben werden erfindungsgemäß durch den Extrakt gemäß den Patentansprüchen 1 bis 8, das Verfahren gemäß den Patentansprüchen 9 bis 21 und der pharmazeutischen Zubereitung gemäß dem Patentanspruch 22 gelöst.

Die vorliegende Erfindung beruht unter anderem auf dem überraschenden Befund, daß durch bestimmte antioxidative und/oder sauerstoffbindende Stabilisatoren bzw. Reduktionsmittel, die in der Lage sind, Oxidantien wie z.B. Radikale, Peroxide, Luftsauerstoff etc. im Extrakt abzubauen und/oder den Abbau von Hyperforin zu hemmen, und gegebenenfalls Durchführung der Extraktion unter einem Inertgas wie Stickstoff und/oder Lichtausschluß und/oder eines in seinem Gehalt an freiem Sauerstoff stark verminderten Lösungsmittels, der so erhaltene Extrakt gegenüber einem unbehandelten Hypericumextrakt wesentlich länger stabil bleibt. Dieser Extrakt kann im Gegensatz zu den Beobachtungen von R. Berghöfer und J. Hölzl, loc.
cit. auch aus einer getrockneten, gelagerten Droge stammen. Ein in seinem Sauerstoffgehalt stark vermindertes Lösungsmittel kann durch physikalische Behandlung, z.B. Spülen mit einem Inertgas wie Stickstoff, hergestellt werden. Wird ein Hypericumextrakt erfindungsgemäß konserviert bzw. stabilisiert, insbesondere durch Zusatz eines Antioxidans und gegebenenfalls unter Ausschluß von Licht und Luftsauerstoff hergestellt, bleibt das Hyperforin in diesem Extrakt praktisch stabil. Der Schutz vor Licht und Luftsauerstoff kann auch durch eine entsprechende pharmazeutische Formulierung erreicht werden.

In einer bevorzugten Ausführungsform des erfindungsgemäßen Verfahrens zur Herstellung des stabilisierten Extraktes wird die frische oder vorzugsweise getrocknete Johanniskrautdroge mit wäßrigem Ethanol extrahiert, dessen Gehalt an freiem Sauerstoff durch physikalische Behandlung stark vermindert wurde. Der Extraktlösung wird entsprechend gegebenenfalls vorliegenden Oxidantien, ein Antioxidationsmittel als Stabilisator zugesetzt und darin gelöst. Weitere Beispiele für bevorzugte Lösungsmittel zur Extraktion von Johanniskraut sind wäßriges Methanol, Alkane mit niedrigen Siedepunkten mit etwa 5 bis 8 Kohlenstoffatomen, wie Pentane, Hexane und Heptane, insbesondere n-Heptan und flüssiges oder überkritisches Kohlendioxid. Der Ausdruck "wäßriges Methanol oder Ethanol" bedeutet Methanol oder Ethanol mit einem Wassergehalt von vorzugsweise bis zu etwa 40 Vol.%.

Spezielle Beispiele für bevorzugte antioxidative Stabilisatoren bzw. Antioxidationsmittel sind pharmakologisch verträgliche Substanzen, die in der Lage sind, den Abbau von Hyperforin zu hemmen und/oder im Extrakt oder Arzneimittel enthaltende Oxidantien zu reduzieren. Es sind dies insbesondere organische Thiolverbindungen, wie Cystein und Glutathion, sowie Ascorbinsäure und Derivate dieser Verbindungen wie die Fettsäureester der Ascorbinsäure, z.B. das Myristat, Palmitat und Stearat.

Die antioxidativen Stabilisatoren werden in einer zur Stabilisierung des Hyperforins ausreichenden Menge dem Hypericumpräparat einverleibt. Im allgemeinen genügen Konzentrationen von 0,01 bis 5 % antioxidativer Stabilisator, bezogen auf den Hypericumextrakt.

In einer anderen Ausführungsform wird ebenfalls wie vorstehend beschrieben verfahren, der Zusatz des Stabilisators aber erst nach der Trocknung der Extraktlösung ausgeführt. In einer weiteren bevorzugten Ausführungsform wird der antioxidative Stabilisator erst auf der Stufe der fertigen Arzneiform zusammen mit den anderen pharmazeutischen Hilfsstoffen zugesetzt.

Vorzugsweise werden alle Ausführungsformen unter Licht- und Sauerstoffausschluß durchgerührt.
Die erhaltenen Extrakte können zusammen mit üblichen pharmazeutischen Hilfsstoffen, gegebenenfalls nach erneutem Zusatz eines Stabilisators, zu pharmazeutischen Zubereitungen wie Kapseln, Filmtabletten und Dragees verarbeitet werden.

Als pharmazeutische Hilfsstoffe werden übliche Füll-, Binde-, Spreng-, Schmier- und Überzugsmittel für Filmtabletten und Dragees sowie Öle und Fette als Füllmassen für Weichgelatinekapseln verwendet.

Die Erfindung wird anhand folgender Beispiele weiter erläutert. Prozentangaben beziehen sich auf das Gewicht, sofern nichts anderes angegeben ist. Als Inertgas (Schutzgas) wurde Stickstoff verwendet. Es kann jedoch auch ein anderes Inertgas wie Argon oder Krypton verwendet werden.

### Beispiel (Vergleich)

1 kg Johanniskrautdroge wurden in einer Mühle fein gemahlen und mit 7 kg 70(v/v)%-igem Ethanol versetzt. Die Suspension aus 1 kg Droge und 7 kg Lösungsmittel wurde eine Stunde bei 55°C unter Inertgas intensiv gerührt. Sodann wurde der erhaltene Extrakt von der Droge mittels einer Zentrifuge getrennt. Der Drogenrückstand wurde ein zweites Mal in gleicher Weise mit 7 kg Lösungsmittel extrahiert. Die beiden Extraktlösungen wurden vereinigt, und mit einem Aliquot wurde der Trockenrückstand im Extrakt bestimmt. Der Extrakt wurde schonend unter vermindertem Druck auf einen Trockenrückstand von 72 % konzentriert und bei 40°C im Vakuum unter vermindertem Druck nachgetrocknet. Es wurden 0,44 kg Trockenextrakt erhalten. Der Gehalt an Hyperforin betrug 0,37 %.

### Beispiel 2

8 kg Johanniskrautdroge wurden in einer Mühle fein gemahlen und mit 56 kg 70(v/v)%-igem Ethanol versetzt. Zuvor wurde das eingesetzte Lösungsmittel mittels Inertgas-Spülen in seinem Sauerstoffgehalt vermindert. Die Suspension aus 8 kg Droge und 56 kg Lösungsmittel wurde eine Stunde bei 55°C unter Inertgas intensiv gerührt. Sodann wurde der erhaltene Extrakt von der Droge mittels einer Zentrifuge unter Inertbegasung mit Stickstoff als Inertgas getrennt. Der Drogenrückstand wurde ein zweites Mal in gleicher Weise extrahiert. Die beiden Extraktlösungen wurden vereinigt und mit 0,1% Ascorbinsäure versetzt. Die Lösung wurde 10 min intensiv unter Stickstoff als Inertgas gerührt und anschließend schonend unter vermindertem Druck auf einen Trockenrückstandsgehalt von 70 % konzentriert und bei 40°C unter vermindertem Druck nachgetrocknet. Es wurden 2,52 kg stabilisierter Trockenextrakt mit einem Hyperforingehalt von 0,9 % erhalten.

### Beispiel 3

400 g trockenes, fein gemahlenes Johanniskraut wurden mit 3,2 kg n-Heptan versetzt. Der Mischung wurden 12 mg Ascorbinsäurepalmitat zugegeben und dann während 1 Stunde unter ständigem Rühren bei 50 °C unter Lichtausschluss extrahiert. Anschließend wurde über ein Seitz Supra 1500 Filter abgesaugt und der Drogenrückstand noch ein zweites Mal in der gleichen Weise extrahiert. Die vereinigten Extraktlösungen wurden am Rotationsverdampfer bei 35 °C unter Lichtausschluß auf einen Trockenrückstandsgehalt von ca. 70 % konzentriert und schließlich gefriergetrocknet. Es resultierten 21 g Trockenextrakt mit einem Gehalt an Hyperforin von 1,7 %.

### Beispiel 4

470 g trockenes, fein gemahlenes -Johanniskraut wurden mit 15 mg Ascorbinsäurepalmitat versetzt, in eine Hochdruckextraktionsanlage gegeben und bei 200 bar bei 35 °C extrahiert. Es wurden etwa 7,5 kg Kohlendioxid eingesetzt. Nach der Extraktion wurde der Druck auf 60 bar reduziert zwecks Abscheidung des Extraktes. Der Extrakt wurde der Apparatur entnommen und mit 10 mg Ascorbinsäurepalmitat vermischt. Es resultierten 12,2 g Trockenextrakt mit einem Gehalt an Hyperforin von 1,9 %.

### Beispiel 5

2,4 kg Johanniskrautdroge wurden in einer Mühle fein gemahlen und mit 16 kg 80(v/v)%-igem Methanol versetzt, das vorher mit Stickstoff durchgespült worden war. Dieses Gemisch wurde anschließend eine Stunde bei 55 °C gerührt. Die erhaltene Extraktionslösung wurde durch Zentrifugieren vom Drogenrückstand getrennt. Der Rückstand wurde nochmals in der gleichen Weise extrahiert. Beide Extraklösungen wurden vereinigt und mit 1,0 Gew-% Ascorbinsäure versetzt. Diese Lösung wurde während 15 Minuten gerührt. Dann wurde die Extraktlösung schonend unter vermindertem Druck eingeengt auf einen Trockenrückstandsgehalt von 70 %. Danach wurde bei 40 °C unter vermindertem Druck nachgetrocknet. Es resultierten 0,5 kg stabilisierter Trockenextrakt mit einem Gehalt an Hyperforin von 0,7 %. Der Gesamthypericingehalt in diesem Extrakt betrug 0,46 %.

### Beispiel 6

### Prüfung der Hyperforin-Stabilität

In diesem Beispiel wurde der Hyperforingehalt eines Extraktes gemäß Beispiel 1 ohne besondere Vorsichtsmaßnahmen und Zusätze bei der Herstellung mit erfindungsgemäß hergestellten Extrakten aus den Beispielen 2-5 verglichen. Die erfindungsgemäß hergestellten Extrakte wurden unter Stickstoff und Ausschluß von Licht bei Raumtemperatur gelagert. Die Ergebnisse sind in Tabelle I zusammengefaßt. Das Ergebnis zeigt bei den erfindungsgemäß hergestellten Extrakten einen nach 6 Monaten unveränderten Hyperforingehalt.

**Tabelle I**

| Trockenextrakt | Hyperforin | | Hyperforingehalt % |
|---|---|---|---|
| Beispiel | Ausgangsgehalt % | nach 13 Wochen | nach 6 Monaten |
| Beispiel 1 | 0,37 | 0,0 | 0,0 |
| Beispiel 2 | 0,9 | 0,9 | 0,88 |
| Beispiel 3 | 1,7 | 1,7 | 1,68 |
| Beispiel 4 | 1,9 | 1,9 | 1,89 |
| Beispiel 5 | 0,7 | 0,7 | 0,63 |

### Beispiel 7

### Weichgelatinekapseln mit Hypericumextrakt

| Zusammensetzung | |
|---|---|
| Hypericumtrockenextrakt | 300 mg |
| Ascorbinsäure | 0,25 mg |
| Octyldodecanol | 200 mg |

### Herstellung:

Trockenextrakt und Ascorbinsäure wurden zusammen in Octyldodecanol dispergiert und unter Ausschluß von Luftsauerstoff zu Weichgelatinekapseln verarbeitet.

### Beispiel 8

### Filmtablette mit Hypericumextrakt

| Zusammensetzung | |
|---|---|
| Hypericum-Trockenextrakt | 300 mg |
| Cellulose | 100 mg |
| modifizierte Stärke | 90 mg |
| Na-Carboxymethylcellulose | 30 mg |
| hochdisperses Silicumdioxid | 5,0 mg |
| Ascorbinsäure | 5,0 mg |
| Magnesiumstearat | 5,0 mg |
| Hydroxypropylmethylcellulose-Überzug | 20,0 mg |

### Herstellung:

Die Bestandteile wurden in einem Mischer trocken gemischt und direkt zu Tabletten verpreßt. Die erhaltenen Tabletten wurden mit einem Überzug aus Hydroxypropylmethylcellulose beschichtet.

## Patentansprüche

1. Stabiler Extrakt aus Hypericum perforatum L. (Johanniskraut) mit einem Gehalt von 0,1% bis 2% Hyperforin, ausgenommen 2% Hyperforin, **dadurch gekennzeichnet, daß** das Hyperforin durch einen Stabilisator aus der Gruppe bestehend aus antioxidativen und sauerstoßbindenden Stabilisatoren bzw. Reduktionsmitteln gegen Zerfall oder Abbau stabilisiert ist.

2. Stabiler Extrakt nach Anspruch 1, **gekennzeichnet durch** einen Gehalt eines Stabiliators aus der Gruppe bestehend aus organischen Thiolverbindungen und Ascorbinsäure und deren Derivaten in einer zur Stabilisierung des Hyperforins ausreichenden Menge.

3. Stabiler Extrakt nach Anspruch 1 oder, 2, **dadurch gekennzeichnet, daß** der Stabilisator in einer Konzentration von 0,01% bis 5%, bezogen auf den Extrakt, vorliegt.

4. Stabiler Extrakt nach Anspruch 1 oder 2, **dadurch gekennzeichnet, daß** der Stabilisator in einer Konzentration von 0,2% bis 1%, bezogen auf den Extrakt, vorliegt.

5. Stabiler Extrakt nach einem der Ansprüche 1 bis 4, **dadurch gekennzeichnet, daß** der Stabilisator Cystein ist.

6. Stabiler Extrakt nach einem der Ansprüche 1. bis 4, **dadurch gekennzeichnet, daß** der Stabilisator Glutathion ist.

7. Stabiler Extrakt nach einem der Ansprüche 1 bis 4, **dadurch gekennzeichnet, daß** der Stabilisator Ascorbinsäure ist.

8. Stabiler.Extrakt nach einem der Ansprüche 1 bis 4, **dadurch gekennzeichnet, daß** der Stabilisator ein Fettsäureester der Ascorbinsäure ist.

9. Verfahren zur Herstellung eines stabilen Extraktes aus Hypericum perforatum L. (Johanniskraut) mit einem Gehalt von 0,1% bis 2% Hyperforin, ausgenommen 2% Hyperforin, bei dem eine Johanniskraut-Droge mit einem pharmazeutisch üblichen anorganischen oder organischen Lösungsmittel oder dessen Gemischen, ausgenommen ölige Extraktionsmittel, extrahiert wird und bei dem ein Stabilisator aus der Gruppe bestehend aus organischen- Thiolverbindungen, Ascorbinsäure und deren Derivate wahlweise während oder nach der Herstellung des Extraktes in einer zur Stabilisierung des Hyperforins ausreichenden Menge zugegeben wird und aus dem so erhaltenen Flüssigextrakt ein Trockenextrakt gewonnen wird.

10. Verfahren nach Anspruch 9, **dadurch gekennzeichnet, daß** frische Johanniskraut-Droge extrahiert wird.

11. Verfahren nach Anspruch 9, **dadurch gekennzeichnet, daß** getrocknete Johanniskraut-Droge extrahiert wird.

12. Verfahren nach einem der Ansprüche 9 bis 11, **dadurch gekennzeichnet, daß** als Stabilisator Cystein verwendet wird.

13. Verfahren nach einem der Ansprüche 9 bis 11, **dadurch gekennzeichnet, daß** als Stabilisator Glutathion verwendet wird.

14. Verfahren nach einem der Ansprüche 9 bis 11, **dadurch gekennzeichnet, daß** als Stabilisator Ascorbinsäure verwendet wird.

15. Verfahren nach einem der Ansprüche 9 bis 11, **dadurch gekennzeichnet, daß** als Stabilisator ein Fettsäureester der Ascorbinsäure verwendet wird.

16. Verfahren nach einem der Ansprüche 9 bis 15, **dadurch gekennzeichnet, daß** der Stabilisator in einer Konzentration von 0,01% bis 5%, vorzugsweise 0,2% bis 1%, bezogen auf den Extrakt, zugesetzt wird.

17. Verfahren nach einem der Ansprüche 9 bis 16, **dadurch gekennzeichnet, daß** mit einem Lösungsmittel extrahiert wird, dessen Gehalt an freiem Sauerstoff niedrig ist oder stark vermindert worden ist.

18. Verfahren nach einem der Ansprüche 9 bis 17, **dadurch gekennzeichnet, daß** zur Extraktion ein Lösungsmittel aus der Gruppe bestehend aus wäßrigem Ethanol, wäßrigem Methanol; n-Heptan und flüssigem oder überkritischem Kohlendioxid verwendet wird.

19. Verfahren nach einem der Ansprüche 9 bis 18, **dadurch gekennzeichnet, daß** der Stabilisator nach der Trocknung der Extraktionslösung zugesetzt wird.

20. Verfahren nach einem der. Ansprüche 9 bis 19, **dadurch gekennzeichnet, daß** der Stabilisator erst auf der Stufe der fertigen Arzneiform zusammen mit üblichen pharmazeutischen Hilfsstoffen zugesetzt wird.

21. Verfahren nach einem der Ansprüche 9 bis 20, **dadurch gekennzeichnet, daß** das Verfahren unter Licht- und/oder Sauerstoffausschluß durchgeführt wird.

22. Pharmazeutische Zubereitung, enthaltend einen Extrakt nach einem der Ansprüche 1 bis 8 und übliche pharmazeutische Hilfsstoffe zur Behandlung von Depressionen und psychovegetativen Erkrankungen.

## Claims

1. Stable extract of Hypericum perforatum L. (St. John's wort) having a Hyperforin content of 0.1 % to 2 %, 2 % Hyperforin being excluded, **characterized in that** the Hyperforin is stabilized against decomposition or degradation by means of a stabilizer from the group consisting of anti-oxidant and oxygen-binding stabilizers and reducing agents.

2. Stable extract according to claim 1 **characterized by** a content of a stabilizer from the group of organic thiol compounds, ascorbic acid and derivatives thereof in an amount sufficient to stabilize the Hyperforin.

3. Stable extract according to claim 1 or 2 **characterized in that** the stabilizer is present in a concentration of 0.01 % to 5 % based on the extract.

4. Stable extract according to claim 1 or 2 **characterized in that** the stabilizer is present in a concentration of 0.2 % to 1 % based on the extract.

5. Stable extract according to any one of claims 1 to 4 **characterized in that** the stabilizer is cysteine.

6. Stable extract according to any one of claims 1 to 4 **characterized in that** the stabilizer is glutathione.

7. Stable extract according to any one of claims 1 to 4 **characterized in that** the stabilizer is ascorbic acid.

8. Stable extract according to any one of claims 1 to 4 **characterized in that** the stabilizer is a fatty acid ester of ascorbic acid.

9. Process for the preparation of a stable extract of Hypericum perforatum L. (St. John's wort) having a Hyperforin content of 0.1% to 2 %, 2 % Hyperforin being excluded, wherein a drug of St. John's wort is extracted with a pharmaceutically conventional inorganic or organic solvent or mixtures thereof, with the proviso that the solvents are no oily extraction agents and wherein a stabilizer from the group consisting of organic thiol compounds, ascorbic acid and derivatives thereof is added, optionally during or after the preparation of the extract, in an amount sufficient to stabilize the Hyperforin and wherein a dry extract is obtained from the thus obtained liquid extract.

10. Process according to claim 9 **characterized in that** a fresh drug of St. John's wort is extracted.

11. Process according to claim 9 **characterized in that** a dried drug of St. John's wort is extracted.

12. Process according to any one of claims 9 to 11 **characterized in that** cysteine is used as stabilizer.

13. Process according to any one of claims 9 to 11 **characterized in that** glutathione is used as stabilizer.

14. Process according to any one of claims 9 to 11 **characterized in that** ascorbic acid is used as stabilizer.

15. Process according to any one of claims 9 to 11 **characterized in that** a fatty acid ester of ascorbic acid is used as stabilizer.

16. Process according to any one of claims 9 to 15 **characterized in that** the stabilizer is added in a concentration of 0.01 % to 5 %, preferably 0.2 % to 1 % based on the extract.

17. Process according to any one of claims 9 to 16 **characterized in that** the extraction is carried out with a solvent having a content of free oxygen that is low or has been significantly reduced.

18. Process according to any one of claims 9 to 17 **characterized in that** for the extraction a solvent from the group consisting of aqueous ethanol, aqueous methanol n-heptane and liquid or supercritical carbon dioxide is used.

19. Process according to any one of claims 9 to 18 **characterized in that** the stabilizer is added after drying the extract solution.

20. Process according to any one of claims 9 to 19 **characterized in that** the stabilizer is added at the stage of the finished pharmaceutical composition together with conventional pharmaceutical additives.

21. Process according to any one of claims 9 to 20 **characterized in that** the process is carried out under the exclusion of light and/or oxygen.

22. Pharmaceutical composition containing an extract according to any one of claims 1 to 8 and conventional pharmaceutical additives for the treatment of depressions and psychovegetative disorders.

## Revendications

1. Extrait stable *d'Hypericum perforatum L.* (millepertuis) avec une teneur de 0,1 % à 2 % d'hyperforine, à l'exclusion de 2 % d'hyperforine, **caractérisé en ce que** l'hyperforine est stabilisée contre la destruction ou la dégradation par un stabilisant choisi dans le groupe comprenant des stabilisants anti-oxydants et de liaison à l'oxygène et des réducteurs.

2. Extrait stable selon la revendication 1, **caractérisé par** une teneur en un stabilisant choisi dans le groupe comprenant les groupements thiol organiques et l'acide ascorbique et leurs dérivés en une quantité suffisante pour la stabilisation de l'hyperforine.

3. Extrait stable selon la revendication 1 ou la revendication 2, **caractérisé en ce que** le stabilisant est présent en une concentration de 0,01 % à 5 % de l'extrait.

4. Extrait stable selon la revendication 1 ou la revendication 2, **caractérisé en ce que** le stabilisant est présent en une concentration de 0,2 % à 1 % de l'extrait.

5. Extrait stable selon l'une des revendications 1 à 4, **caractérisé en ce que** le stabilisant est la cystéine.

6. Extrait stable selon l'une des revendications 1 à 4, **caractérisé en ce que** le stabilisant est le glutathion.

7. Extrait stable selon l'une des revendications 1 à 4, **caractérisé en ce que** le stabilisant est l'acide ascorbique.

8. Extrait stable selon l'une des revendications 1 à 4, **caractérisé en ce que** le stabilisant est un ester d'acide gras de l'acide ascorbique.

9. Procédé de fabrication d'un extrait stable *d'Hypericum perforatum L.* (millepertuis) avec une teneur de 0,1 % à 2 % d'hyperforine, à l'exclusion de 2 % d'hyperforine, selon lequel une drogue de millepertuis est extraite avec un solvant inorganique ou organique pharmaceutique habituel ou avec leurs mélanges, à l'exception des agents d'extraction huileux, et selon lequel un stabilisant choisi dans le groupe comprenant les groupements thiol organiques, l'acide ascorbique et leurs dérivés est ajouté optionnellement pendant ou après la fabrication de l'extrait en une quantité suffisante pour la stabilisation de l'hyperforine et selon lequel un extrait sec est obtenu à partir de l'extrait liquide ainsi obtenu.

10. Procédé selon la revendication 9, **caractérisé en ce que** la drogue de millepertuis fraîche est extraite.

11. Procédé selon la revendication 9, **caractérisé en ce que** la drogue de millepertuis sèche est extraite.

12. Procédé selon l'une des revendications 9 à 11, **caractérisé en ce qu'**on utilise la cystéine comme stabilisant.

13. Procédé selon l'une des revendications 9 à 11, **caractérisé en ce qu'**on utilise le glutathion comme stabilisant.

14. Procédé selon l'une des revendications 9 à 11, **caractérisé en ce qu'**on utilise l'acide ascorbique comme stabilisant.

15. Procédé selon l'une des revendications 9 à 11, **caractérisé en ce qu'**on utilise un ester d'acide gras de l'acide ascorbique comme stabilisant.

16. Procédé selon l'une des revendications 9 à 15, **caractérisé en ce que** le stabilisant est ajouté en une concentration de 0,01 % à 5 %, de préférence de 0,2 % à 1 % de l'extrait.

17. Procédé selon l'une des revendications 9 à 16, **caractérisé en ce que** l'extraction est réalisée avec un solvant dont la teneur en oxygène libre est faible ou fortement réduite.

18. Procédé selon l'une des revendications 9 à 17, **caractérisé en ce que**, pour l'extraction, on utilise un solvant choisi dans le groupe comprenant l'éthanol aqueux, le méthanol aqueux, le n-heptane et le dioxyde de carbone fluide ou supercritique.

19. Procédé selon l'une des revendications 9 à 18, **caractérisé en ce que** le stabilisant est ajouté après séchage de la solution d'extraction.

20. Procédé selon l'une des revendications 9 à 19, **caractérisé en ce que** le stabilisant n'est ajouté qu'au stade de forme médicamenteuse finie avec les excipients pharmaceutiques habituels.

21. Procédé selon l'une des revendications 9 à 20, **caractérisé en ce que** le procédé est mis en oeuvre à l'abri de la lumière et/ou de l'oxygène.

22. Préparation pharmaceutique contenant un extrait selon l'une des revendications 1 à 8 et des excipients pharmaceutiques habituels pour le traitement de la dépression et de maladies psycho-végétatives.
